# EUROPEAN PATENT APPLICATION

(11) **EP 2 172 545 A1**
(43) Date of publication of application: **07.04.2010**
(21) Application number: 08164983.2
(22) Date of filing: 24.09.2008
(51) Int. Cl.: C12N 9/80

(54) **Preparation of purified peptide deformylase**

(71) Applicant: DSM IP Assets B.V., 6411 TE Heerlen (NL)
(72) Inventor: The designation of the inventor has not yet been filed
(74) Representative: Hoogendam, Gerrie Christine

(57) **Abstract**

The invention relates to a method for preparing a purified peptide deformylase, comprising contacting a liquid which comprises a peptide deformylase containing an ionic tag and at least one other enzyme with an ion exchange material to which material the ionic tag binds by ionic interaction and separating the other enzyme from said peptide deformylase. The invention further provides a peptide deformylase comprising an ionic tag, a vector comprising a nucleic acid sequence encoding such a peptide deformylase and a cell comprising such a vector.

## Description

The invention relates to a method for preparing a purified peptide deformylase, to a novel peptide deformylase, to a cell capable of expressing a peptide deformylase, to a vector comprising a nucleic acid sequence encoding a peptide deformylase and to the use of the peptide deformylase of the invention.

Peptide deformylase (PDF) is regarded as an attractive biocatalyst for the synthesis of enantiomerically pure amino acid derivatives or for catalysing the deprotection of peptides which are protected with a formyl group at the N-terminal alpha-amino function, *e.g*. in peptide synthesis (Sonke et al. Journal of Molecular Catalysis B: Enzymatic 29 (2004) 265-277). Sonke *et al.* also report that PDF that has not been sufficiently purified from a cell from which it is recovered may cause extensive peptidic bond hydrolysis of a peptide of which the N-terminal amino group is protected with a formyl group or which has been deprotected using PDF, possibly as a result of peptidase activity originating from the cells used for expressing PDF. Sonke *et al.* describe a recovery and purification process wherein the cells are disintegrated, using a buffer containing 100 mM KF. After centrifugation, polyethylene amine is added to the supernatant (containing PDF), in order to precipitate nucleic acids. After centrifugation, the supernatant (containing PDF) is applied to a Met-Lys Sepharose™ affinity chromatography column that has been equilibrated with a buffer containing 100 mM KF, which is needed for the binding of the PDF to the column. Then the column is washed with buffer to remove non-binding proteins. Thereafter PDF is eluted with buffer containing KCI. Finally, PDF is concentrated using ultrafiltration.

The described method for purifying PDF is in practice only suitable for use on a laboratory scale. Met-Lys modified Sepharose™ is not readily commercially available, at least not on a large scale. Further, the preparation of this material involves a complicated chemical preparation process. Moreover, it is considered that the need for fluoride in the method makes it necessary that special precautions are taken in order to avoid corrosion of equipment used. In the presence of fluoride even enamelcoated reactors and conduits have been noticed to become corroded. Thus, special precautions would have to be taken to carry out such method on an industrial scale. Special equipment would be required, adapted to withstand the detrimental effect of fluoride. Such equipment may not be readily available. The need for such equipment may also add to the complexity of the purification method. Further, it is contemplated that fluoride may have an inhibitory effect on the activity of PDF.

WO 02/02763 relates to an iron complex of *S. Aureus* peptide deformylase having an N-terminal and/or a C-terminal affinity tag, such as an oligohistidine tag. The isolation of the tagged PDF complex using immobilised-metal affinity chromatography or metal-chelate chromatography using an imidazole solution is described. Imidazole is undesired for use on an industrial scale, because of its corrosive character. Further, the column materials for immobilised-metal affinity chromatography or metal-chelate chromatography may not be readily available for large scale preparation of the PDF. Furthermore, an isolation method in order to obtain a PDF composition with a reduced peptidase activity towards formyl N-terminal protected peptides and/or formyl N-terminal deprotected peptides is not suggested.

It is an object of the present invention to provide a novel method to obtain a PDF, that may serve as an alternative to the above described methods.

It is in particular an object to provide a method wherein a PDF is obtained which is essentially free of components that cause peptidic bond hydrolysis, or wherein at least the activity with respect to peptidic bond hydrolysis is relatively low compared to a crude enzyme preparation obtained from an organism producing PDF.

It is in particular an object of the present invention to provide a method for preparing a purified PDF, in particular a purified PDF suitable for use in the preparation of peptides or peptide derivatives or in peptide synthesis, which purification method is relatively simple to be employed, also on an industrial scale,

It is in particular an object of the invention to provide a method for preparing a purified PDF, wherein the risk of corrosion or another detrimental effect of equipment used is reduced, also if standard equipment for enzyme purification is used.

It is further an object to provide a novel PDF that can be used as an alternative to known PDF, in particular for use in the preparation of a peptide or peptide derivative.

One or more other objects that are met by the present invention will be apparent from the description, below.

It has now been found possible to prepare purified PDF, suitable in particular for use in the preparation of amino-acid derivatives, peptide derivatives or in peptide synthesis, also in the absence of an added corrosive substance, such as added fluoride (*e.g*. KF) and/or imidazole.

Accordingly, the present invention provides a number of methods, which are linked by the general inventive concept that they provide alternative solutions to the same problem, identified by the present inventors, in particular the provision of a method that can be carried out in relatively simple equipment, also on an industrial scale, also in the absence of a corroding or otherwise detrimental amount of fluoride, imidazole or other corrosive substance.

In an embodiment, the invention relates to a method for preparing a purified peptide deformylase, comprising contacting a liquid which comprises a peptide deformylase containing an ionic tag and at least one other enzyme with an ion exchange material to which material the ionic tag binds by ionic interaction and separating the other enzyme from said peptide deformylase.

In an embodiment, the invention relates to a method for preparing a purified peptide deformylase, comprising subjecting a liquid which comprises a peptide deformylase and at least one impurity to ultrafiltration, whereby the impurity is retained in the retentate and a filtrate is obtained comprising peptide deformylase.

The invention further relates to a PDF, comprising an internal ionic tag.

The invention further relates to a vector comprising a nucleic acid sequence encoding a peptide deformylase according to the invention.

The invention further relates to a cell comprising a vector comprising a nucleic acid sequence encoding a peptide deformylase according to the invention. The cell may be a host cell selected from bacteria, yeasts or fungi. In particular the host cell may be selected from the genera selected from the group of *Aspergillus, Penicillium, Saccharomyces, Kluyveromyces, Pichia, Candida, Hansenula, Actinoplanes, Bacillus, Corynebacterium, Escherichia, Gluconobacter, Pseudomonas, and Streptomyces.*

It is an advantage that a method according to the invention can be carried out - if desired - without needing to add fluoride (*e.g.* KF), imidazole, or another corrosive, toxic or odorous substance - for instance dithioerythrol, mercaptoethanol or another toxic or odorous thiol which may be needed or at least advised when using immobilised metal affinity chromatography for the separation of proteins - in any method step.

This facilitates the method considerably and allows the method also to be carried out using relatively simple equipment, also at a large scale. In particular, no special precautions need to be taken to protect the equipment from corrosion due to fluoride or another detrimental effect of fluoride. In principle, a trace of fluoride, imidazole or another corrosive substance may be present, *e.g.* as a result of an impurity or as the result of the substance (*e.g.* fluoride) being naturally present in a component used in the production or purification of the PDF, as long as the concentration is low enough to avoid the risk of a substantial corroding effect. For instance, fluoride is naturally occurring in living organisms. Also a salt, *e.g.* a buffer salt, or a solvent used in a method of the invention may comprise some fluoride. For instance, tap water may comprise up to about 1 mg/L fluoride. In view thereof, a method according to the invention is generally carried out in a medium essentially free of fluoride or comprising only a minor amount of fluoride which minor amount can be attributed to fluoride that is naturally present in an organism used for obtaining PDF and/or fluoride that is present as an impurity in a component used in a method of the invention. Usually, the fluoride concentration is less than 10 mg/L. Preferably, the fluoride concentration is about 1 mg/L or less, more preferably about 0.1 mg/L or less.

A purification method according to the invention may be carried out using only aqueous liquids, *e.g.* for lysing cells from which the PDF is isolated, as elution buffer (in a method making use of ion exchange) or as liquid wherein the PDF is dissolved during filtration. A liquid is considered aqueous, if water is the major or only solvent. In particular, a liquid is in particular considered aqueous if the water content is 60-100 wt.%, more in particular 80-100 wt. %, 90-100 wt.% or 99-100 wt.%, based on total liquids. If desired, one or more organic solvents may be present, to form the balance of the liquid.

As used herein, the term "amino acid" includes underivatised amino acids, and amino acid derivatives of which one or more functional groups, in particular at least one carboxylic acid function and/or at least one amino function is protected with a protective group, unless specified otherwise. Amino acid derivatives in particular include amino acid alcohols and amino acid esters.

As used herein, the term "peptide" includes underivatised peptides, and peptide derivatives of which one or more functional groups, in particular at least one carboxylic acid function and/or at least one amino function is protected with a protective group, unless specified otherwise. Peptide derivatives in particular include peptide alcohols and peptide esters.

The term "peptide deformylase" or - abbreviated - "PDF" is used herein to describe an enzyme showing activity with respect to catalysing the removal of a formyl group from a peptide, a peptide derivative, an amino acid or an amino acid derivative of which the N-terminal alpha-amino function is protected with a formyl group. This activity may be determined by a method as described in the examples. Usually, the deformylase activity of a PDF is substantially higher than the deacetylase activity, in particular at least 10 times as high, more in particular at least 100 times as high. The deacetylase activity may be determined in the same manner as the deformylase activity, with the proviso that the formyl group is replacted by an acetyl group.

In particular enzymes classified as belonging to EC 3.5.1.8, EC 3.5.1.10, EC 3.5.1.27, EC 3.5.1.31, EC 3.5.1.68, EC 3.5.1.88 and EC 3.5.1.91 are meant to be included when referring to a PDF. More in particular, a PDF prepared in accordance with the invention may be selected from enzymes from the group of enzymes of EC 3.5.1.27, enzymes of EC 3.5.1.31 and enzymes of EC 3.5.1.88.

In principle, PDF originating from any PDF producing organism may be purified. In particular PDF may originate from a PDF producing organism selected from *Escherichia,* in particular *Escherichia coli; Thermus,* in particular *Thermus thermophilus*; and *Bacillus,* in particular *Bacillus stearothermophilus.* Further, in particular a PDF originating from an organism selected from the group of *Borelia,* in particular *Borrelia burgdorferi; Leptospira,* in particular *Leptospira interrogans,* and *Lactobacilus* in particular *Lactobacillus plantarum* may be purified. When referred to a PDF from a particular source not only PDF actually produced in a wild-type organism but also recombinant PDF, originating from a first organism, but actually produced in a (genetically modified) second organism, is specifically meant to be included as PDF from that first organism. When referred to a PDF from a particular source, this referral also includes mutants of a naturally occurring PDF with PDF activity. Mutants of wild-type PDF can for example be made by modifying the encoding DNA of an organism capable of producing PDF using mutagenesis techniques known to the person skilled in the art (random mutagenesis, site-directed mutagenesis, directed evolution, gene recombination, *etc.*). In particular the DNA may be modified such that it encodes an enzyme that differs by at least one amino acid from the wild-type enzyme, so that it encodes an enzyme that comprises one or more deletions, substitutions and/or insertions compared to the wild-type, or such that the mutants combines sequences of two or more parent enzymes and by effecting the expression of the thus modified DNA in a suitable (host) cell.

A method of the invention is in particular suitable to provide a PDF with a low peptidase activity, *i.e.* having a low activity with respect to hydrolysing an amide bond between two amino acid residues of a peptide. It is however envisaged that a method of the invention may be used to separate another impurity, in particular another type of enzyme from the PDF.

Peptidase activity may be distinghuished in aminopeptidase activity, carboxypeptidase activity and endopeptidase. Preferably, the PDF has low activity with respect to each of these specific peptidase activities. In particular, peptidase activity may be below detection level or at least considerably reduced compared to the liquid before purification. This makes the PDF (obtained in a method) according to the invention particularly suitable for use in peptide synthesis or another application, wherein (a high) peptidase activity, in particular (a high) amino peptidase activity, may have a detrimental effect, such as catalysing undesired hydrolysis of a peptide bond or another amide bond.

In particular in a method of the invention wherein PDF comprising an ionic tag is purified using ion exchange, a purification factor compared to a liquid comprising the PDF prior to purification (usually a cell free extract of a cell culture wherein the PDF has been expressed) of 25 or more, preferably of 250 or more, more preferably of 500 or more, in particular of 1000 or more, *e.g*. up to 2000, in particular of 1500 or less, more in particular of 1400 or less is feasible, if desired. The purification factor as defined herein as the ratio X to Y, wherein X is a specific peptidase activity of liquid, such as an amidase activity, prior to purification divided by a specific PDF activity of the liquid, prior to purification and wherein Y is the specific peptidase activity of the liquid, after purification divided by the specific PDF activity of the liquid, after purification. The amidase activity and the PDF activity can be determined as described in the examples.

As indicated above, in an embodiment PDF to be purified contains a tag. Tags are moieties that increase the affinity of a compound of interest, notably a protein, for a specific chromatographic material. Various types of tags for proteins have been described in the art, as illustrated by K. Terpe in a review published in Appl.Microbiol.Biotechnol. (2003) 60:523-533, and in WO 02/02763, referring to affinity tags for affinity chromatography. Nevertheless, it remains difficult to choose the right purification system for a specific protein of interest, especially if the aim of the purification is to achieve a specific result, such as the removal of one or more components that may cause peptidic bond hydrolysis, whilst maintaining substantial PDF activity.

In accordance with the invention, it has been found that purification is accomplished by providing a PDF with an ionic tag. Ionic tags are charged or chargeable moieties, that contribute to the number of charges of PDF, at least under separation conditions, such that the binding affinity of PDF for a ion exchange material is increased. Surprisingly, this has been found possible, without detrimentally affecting the enzyme activity with respect to deprotecting a formyl protected N-terminal alpha-amino function (as determined by the method given in the examples), or at least not to an unacceptable extent, also if the ionic tag is present in a region of the amino acid sequence of the PDF, remote from its termini. Further, it has surprisingly been found that a host cell comprising a nucleic acid sequence encoding a PDF comprising an ionic tag can be provided that is capable of expressing the tagged PDF with a good yield, comparable to or even higher than the expression of a corresponding wild type PDF. In particular, at least in some embodiments the expression of a PDF comprising an anionic tag (in a part of the enzyme remote from the termini) may be higher than of a PDF comprising an oligohistidine tag, starting at the same amino acid position.

In particular, an ionic tag may comprise an oligopeptide sequence. The ionic tag may thus be a part of the enzyme sequence. Usually, the tag comprises 2-20 amino acid residues. In particular, the number of amino acid residues of the tag may be at least 3, at least 5, at least 6 or at least 7. In particular, the number of amino acid residues of the tag may be 15 or less, 12 or less, 10 or less, or 9 or less.

An ionic tag may in particular comprise either a plurality of amino acid residues having an acidic side chain, or a plurality of amino acid residues having a basic side chain. Under physiological conditions, the former typically is an anionic tag (*i.e.* the acidic side chain will at least predominantly be present as the conjugated base), and the latter a cationic tag (*i.e.* the alkaline side chain will at least predominantly be present as the conjugated acid). Optionally, the tag may further comprise one or more amino acids that are free of an anionic or cationic side-chain. Typically, 30 % or more, preferably 50 % or more, more preferably 75 % or more of the amino acid residues of the tag comprise an anionic or cationic side-chain.

In a specific embodiment all amino acid residues of the ionic tag comprise an anionic or a cationic side-chain.

The position of the ionic tag in the amino acid sequence of the PDF is optionally separated from the position of the native amino acid sequence by a spacer, such as an amino acid sequence of one or more amino acids without a cationic or anionic side-chain.

Anionic side chains (to which may also be referred as 'acidic side chains') are in particular acidic side chains having a pKa (at 25 °C) of about 8 or less, preferably of about 7 or less, more preferably of about 6 or less. Anionic side chains include in particular side chains comprising a carboxylic acid group, such as in glutamate or aspartate residues. When referred to an acid, its corresponding conjugated base, which will generally be the most abundant form of the acid-base pair (such as the carboxylic acid/carboxylate base pair) in the purification method, is meant to be included. Preferred are ionic tags comprising glutamate and/or aspartate residues.

Cationic side chains (to which may also be referred to as 'basic side chains') typically comprise an amine group. When referred to a base (alkaline), its corresponding conjugated acid, which will generally be the most abundant form of the acid-base pair (such as the ammonium/amine base pair) in the purification method, is meant to be included. Typically, amino acid residues providing a side chain comprising an amine group are selected from the group of lysine residues, arginine residues and histidine residues.

In particular a tag may be selected from oligopeptide moieties selected from the group of oligoglutamates, oligoaspartates, oligolysines, oligohistidines and oligoarginines.

In principle, the ionic tag may be provided at any section in the amino acid sequence of the enzyme, provided that the tag is sufficiently exposed at an outer surface of the enzyme, such that it can interact with the ion exchange material. It is further preferred that the tag is provided at such a position of the enzyme that substantial desired enzyme activity is maintained. In particular the tag may be provided at such a position that the desired enzyme activity is at least 60 %, more in particular at least 70 %, even more in particular at least 80 %, of the activity of the corresponding enzyme without the tag. This offers the advantage that the tag will generally not have to be removed for subsequent use.

In principle an ionic tag may be provided at the N-terminal alpha-amino function or the C-terminal carboxylic acid group of the PDF. Such tag is referred herein as an external tag, *i.e.* the amino acid residue at the N-terminus or the C-terminus of the tagged PDF is part of the tag.

In an advantageous embodiment, an ionic tag is present in a part of the enzyme remote from the N-terminus and from the C-terminus, *i.e.* it is an internal tag. It is contemplated that an internal tag is less prone to be released from the enzyme, *e.g.* by undesired hydrolysis, than a tag provided at one of the termini (than an external tag). A preferred start respectively end of the internal ionic tag, depends on the specific enzyme, and may for instance be at least about 5, at least about 20, at least about 40 or at least about 60 amino acid residues from the termini.

The inventors have surprisingly found a region in the amino acid sequence of PDF which can be modified such, that an ionic tag is provided whereby affinity for an ion exchange material is increased, whilst maintaining sufficient activity with respect to formyl deprotection of an N-terminal alpha-amino function protected with a formyl group. In particular, it has been found possible to provide a PDF, wherein an internal ionic tag is present in a flexible loop of the PDF, whilst maintaining substantial peptide deformylase activity. A flexible loop may also be referred to in the art as a "non-conserved region". Preferably the flexible loop, or at least the ionic tag in the flexible loop is exposed to the liquid wherein ion exchange step takes place, such that the PDF is allowed to bind well to the ion exchange material.

*E.g.* in Sequence ID 2, 4 or 6, the ionic tag starts at position 64 of the sequence shown. Such PDF has been found to be purifyable with a method of the invention making use of an ion exchange step and has been shown to have satisfactory deformylase activity. It is contemplated that the tag may be provided starting at a different position in the vicinity of position 64 of an enzyme containing an amino acid sequence as shown in Sequence ID 2, Sequence ID 4, Sequence ID 6, or a homologue thereof for instance at position 60, 61, 62, 63, 65, 66 or 67. In a PDF wherein an amino acid sequence of Sequence ID 2, Sequence ID 4, Sequence ID 6, or a homologue thereof forms part of a longer amino acid sequence, the actual amino acid position at which the tag is provided may be different. In such case the actual position can be based on alignment to PDF from *E. coli* K 12W3110.

An ionic tag comprising a plurality of glutamic acid residues, a plurality of aspartic acid residues or any combination of glutamic acid residues and aspartic acid residues is in particularly suitable for an embodiment of the invention, in particular for an embodiment wherein the tag is an internal tag.

A PDF may be provided with an ionic tag, in particular an oligopeptide sequence, using a molecular biological technique, known *per se.*

Use may be made of one or more mutagenesis techniques, known in the art *per se.* A gene encoding for PDF, *e.g.* PDF from *Escherichia coli , Thermus thermophilus,* or *Bacillus stearothermophilus,* may be modified by replacing, deleting or inserting one or more nucleic acids, in particular an oligonucleotide, encoding an oligopeptide tag, may be inserted at a position of interest. In principle, a part of the tag may include one or more amino acid residues from the wild-type enzyme.

The enzyme may further be modified, *e.g*. to improve one or more of the following aspects: enantioselectivity towards the substrate, activity, stability in one or more solvents, pH profile, temperature profile, substrate profile, susceptibility to inhibition, and substrate-affinity. The enzyme may be provided in a composition, in particular a composition further comprising a catalytic metal ion, such as a catalytic divalent metal ion, in particular Fe(II) or Ni(II), for increased PDF activity.

To the best of the inventors' knowledge, a PDF comprising an internal ionic tag has not been described in the art. Accordingly, the invention also provides a PDF, in particular a recombinant PDF, provided with an internal ionic tag as defined herein, a vector comprising a nucleic acid sequence encoding a peptide deformylase comprising an internal ionic tag and a cell comprising such as vector.

The PDF may in particular be a PDF comprising the following sequence or a homologue of said sequence:
MSVLQVLHIPDERLRKVAKPVEEVNAEIQRIVDDMFETMYAEEGIGLAATQVDIHQRIIV IDVSXₙDRDERLVLINPELLEKSGETGIEEGCLSIPEQRALVPRAEKVKIRALDRDGKPF ELEADGLLAICIQHEMDHLVGKLFMDYLSPLKQQRIRQKVEKLDRLKARA

Herein Xₙ represents the ionic tag wherein n is an integer, usually in the range of 3-20 and each X independently represents an amino acid residue of the tag. Sequence ID 2 and Sequence ID 4 represent a PDF containing an ionic tag containing 3 or 20 amino acid residues, respectively. Sequences only differing from Sequence ID 2 and Sequence ID 4 in that the number of amino acid residues of the tag is different from 3 and 20, in particular sequences only differing from Sequence ID 2 and Sequence ID 4 in that the number of amino acid residues of the tag is from 4 to 19 are in specific homologues of Sequence ID 2 and Sequence ID 4. The same applies *mutatis mutandis* to functional analogues of Sequence ID 1 and Sequence ID 3, see below.

In particular the PDF may be a PDF comprising an amino acid sequence as shown in Sequence ID 2, Sequence ID 4, Sequence ID 6 or a homologue thereof.

A nucleic acid sequence encoding a PDF of the invention may for instance comprise the following sequence:

ATGTCCGTGCTTCAAGTGTTACATATTCCGGACGAGCGGCTTCGCAAAGTTGCTA
AACCGGTAGAAGAAGTGAATGCAGAAATTCAGCGTATCGTCGATGATATGTTCGA
GACGATGTACGCAGAAGAAGGTATTGGCCTGGCGGCAACCCAGGTTGATATCCAT
CAACGTATCATTGTTATTGATGTTTCG(NNN)3nGACCGTGACGAACGGCTAGTGTTA
ATCAATCCAGAGCTTTTAGAAAAAGCGGCGAAACAGGCATTGAAGAAGGTTGCC
TGTCGATCCCTGAACAACGTGCTTTAGTGCCGCGCAGAGAAAGTTAAAATTCG

CGCCCTTGACCGCGACGGTAAACCATTTGAACTGGAAGCAGACGGTCTGTTAGCC
ATCTGTATTCAGCATGAGATGGATCACCTGGTCGGCAAACTGTTTATGGATTATCT
GTCACCGCTGAAACAACAACGTATTCGTCAGAAAGTTGAAAAACTGGATCGTCTG
AAAGCCCGGGCTTAA

Herein each '(NNN)' independently is a codon encoding an amino acid X (see above) and n is an integer, usually in the range of 3-20.

A nucleic acid sequence encoding the PDF of Sequence ID No 2 is given in Sequence ID No 1. A nucleic acid sequence encoding the PDF of Sequence ID No 4 is given in Sequence ID No 3. A nucleic acid sequence encoding the PDF of Sequence ID No 6 is given in Sequence ID No 5.

A vector respectively cell according to the invention may in particular comprise a nucleic acid comprising a sequence according to the sequence of Sequence ID No 1, a sequence according to the sequence of Sequence ID No 3, a sequence according to the sequence of Sequence ID No 5 or a functional analogue thereof. More in particular, the vector may comprise a nucleic acid having the sequence given in Sequence ID No 3 or a functional analogue thereof. In a sequence that is a functional analogue of the sequence of Sequence ID No 1 or Sequence ID No 3, the number of variable codons (NNN) preferably is at least 6. Preferably at least 50 % of the codons encode an amino acid comprising either an carboxylic acid side group or an amino side group.

Amongst the homologues of Sequence ID 2, Sequence ID 4 or Sequence ID 6, are in particular included functional analogues of the PDF, which differ from Sequence ID 2, Sequence ID 4 or Sequence ID 6 respectively, in that up to 6, more in particular up to 4, even more in particular up to 2 amino acids have been deleted and/or wherein up to 10, up to 6 or up to 4 amino acids have been inserted in the region between from the 64^{th} to the 73^{rd} amino acid.of the wild type E. *coli* PDF.

Further, homologues include enzymes comprising mutations in particular in a non-conserved part of the sequence, *e.g*. for the reasons given above. Accordingly, homologues are in particular PDFs having an amino acid sequence identity of at least 60%, preferably at least 65%, more preferably at least 70 %, more preferably at least 75%, more preferably at least 80%, in particular at least 85 %, more in particular at least 90 %, at least 91 %, at least 92 %, at least 93 %, at least 94 %, at least 95 %, at least 96 %, at least 97 %, at least 98 % or at least 99 % with Sequence ID 2, Sequence ID 4 or Sequence ID 6.

For purpose of the present invention, amino acid sequence identity is determined in sequence alignment studies using ClustalW, version 1.82, (http://www.ebi.ac.uk/clustalw) multiple sequence alignment software at default settings (Protein Gap Open Penalty = 10.0; Protein Gap Extension Penalty = 0.2; Protein matrix = Gonnet; Protein ENDGAP = -1; Protein GAPDIST = 4.

Regarding the separation conditions for an embodiment wherein use is made of an ion exchange material to purify a PDF containing an ionic tag , in principle any ion exchange material can be used, with the proviso that a cation exchange material is typically used for a PDF comprising a cationic tag and an anion exchange material for a PDF comprising an anionic tag. The exchange material may be a strong ion exchange material or a weak ion exchange material. Such materials are commercially readily available, *e.g.* from GE Healthcare (*e.g.* Mono Q) or Sigma-Aldrich (*e.g.* Q-sepharose, DEAE-sepharose).

Examples of suitable ion exchange materials may *e.g.* comprise any of the materials selected from agarose, sepharose, cellulose, silica, and polyacrylamide that are derivatised to carry ionisable groups.

Weak anion exchange materials, are *e.g*. weak anion exchange materials comprising tertiary amine groups, such as dialkylamino groups, specific examples of these materials comprise diethylaminoethyl (DEAE) groups, dimethylaminomethyl (DMAE) groups or the like .

Strong anion exchange materials are *e.g.* materials derivatised with quaternary ammonium groups such as trimethyl ammoniumethyl (TMAE) or quaternary amino ethyl.

An example of a weak cation exchange material is a material comprising carboxymethyl groups.

Examples of strong cation exchange materials are materials comprising sulphopropyl groups and materials comprising methyl sulphonate groups.

The conditions can be chosen based on common general knowledge and the information disclosed herein. Typically, first conditions are chosen such that PDF adsorbed to the ion exchange material, *e.g.* adsorbed on a chromatographic column comprising the ion exchange material. Thus, one or more impurities not adsorbed (or not as strongly as the PDF) may be separated from the adsorbed PDF. Thereafter, the PDF is recovered from the ion exchange material by changing conditions such that PDF is no longer adsorbed (or at least less strongly), *e.g.* by contacting the ion exchange material to which the PDF is absorbed with a liquid causing the electrostatic interaction between PDF and the ion exchange material to be reduced, *e.g.* with a liquid having a pH at which ion exchange material charges and/or PDF opposite charges are reduced, with a liquid having an increased ionic strength, such that the electrostatic interaction is reduced, and/or with a liquid comprising ions having a stronger electrostatic interaction with the ion exchange material than the PDF has with the ion exchange material, thereby desorbing PDF. In particular, a high ionic strength liquid may be used, such as an aqueous liquid comprising one ore more salts in a total concentration between 0.25 M and the saturated concentration, more in particular from 0.5 to 2 M. Suitable salts may in particular be selected from the group of chloride salts, sulphate salts, formate salts, acetate salts, citrate salts and nitrate salts of alkaline metals or ammonium, more in particular NaCl, KCI, LiCl, ammonium sulphate, sodium acetate, ammonium acetate and ammonium formate. Thus, PDF may also be purified from one or more impurities that have a stronger electrostatic interaction with the ion exchange material.

Preferably, the PDF is eluted from the ion exchange material using gradient elution, wherein the salt concentration is gradually or stepwise increased.

In particular for binding PDF to the ion exchange material, conditions are chosen such that both the tag and the ion exchange materials are sufficiently ionised and the enzyme is not irreversibly inactivated. For instance, a purification using an anion exchange material may, *e.g.,* be accomplished at a neutral or slightly alkaline pH (*e.g.* at a pH in the range of about 7 to about 8). In case the ionic tag is not sufficiently ionised, the pH can be raised to increase the ionisation degree of the tag, *e.g*. to a pH in the range of about 9 to about 10, provided that the enzyme does not become irreversibly inactivated to an unacceptable extent. For instance, a purification using a cation exchange material may, *e.g.,* be accomplished at a neutral or slightly acidic pH (*e.g.* at a pH in the range of about 5 to about 7), although a higher or lower pH may be used. In case the ionic tag is not sufficiently ionised, the pH can be lowered to increase the number of cationically charged groups in the tag, *e.g.* to about pH 4, provided that the enzyme does nor become irreversibly inactivated.

As indicated above, in an embodiment ultrafiltration is used to prepare a purified PDF. Such PDF may be without a tag, although in principle, the PDF may have been provided with a tag.

Ultrafiltration offers a simple way of separating PDF, including wild-type PDF, from peptidases and the like. Ultrafiltration is an often used technique for concentrating a product of interest, wherein the product of interest is recovered as the retentate. However, the inventors realised that it is also possible to effectively purify the PDF in a liquid, by filtrating the liquid such that a filtrate is obtained comprising the PDF, whereas an undesired enzymatic impurity, in particular a peptidase, is largely retained in the retentate. A purification factor of at least about 25 (compared to cell free extract yet to be purified), in particular of about 30-200 has been found possible, with an acceptable yield.

The cut-off value of the filter is in general chosen between the molecular weight of the PDF and the molecular weight of undesirable macromolecular impurities, notably peptidases. As is clear to the skilled person factors such as the shape of the PDF and the macromolecular compound and/or the formation of supramolecular complexes may be taken into account when determining a particularly suitable filter.

Preferably, the cut-off value is chosen at least twice as high as the molecular weight of the PDF to be purified, more preferably at least about three times as high, even more preferably at least about four times as high. Surprisingly, for a particularly good result, the cut-off value of the filter is preferably relatively high.

The upper-limit is in particular determined by the molecular weight of the macromolecular impurity that is to be separated from the PDF. For instance, the cut-off may be up to ten times the molecular weight, up to seven times the molecular weight of the PDF or up to five times the weight of the PDF.

For instance, the cut-off of the filter may be at least 25 kDa, in particular at least 50 kDa, for a relatively small PDF, having about 300 amino acid residues or less. Preferably the cut-off is more than 70 kDa, or more than 80 kDa. In particular, good results have been achieved with a filter having a molecular weight cut-off of about 100 kDa. This is about 3.5 to about 5 times above the molecular weight of PDF (from *E. coli*). It is contemplated that at a cut-off value above 100 kDa, the yield may be further improved. On the other hand, the cut-off value should be low enough to avoid undesired macromolecular compound(s) to pass through to a undesirable extent. Accordingly, the cut-off should usually be less than about 200 kD, in particular about 150 kDa or less, more in particular about 130 kDa or less, preferably about 120 kDa or less, more preferably about 110 kDa or less.

The invention will now be illustrated by the following examples.

### EXAMPLES

### PDF deformylation activity measurement

Deformylase activity of PDF was routinely measured at 30°C in a total volume of 500 µL. 50 µL of different dilutions of PDF in 20 mM MOPS/NaOH, 100 mM NaCl, pH 7.7, 10 µg/mL bovine liver catalase (Sigma), and 2 mM TCEP, were mixed with 450 µL of substrate solution composed of 110 mM MOPS/NaOH buffer (pH 7.2), 300 mM NaCl, 0.1 mg/mL bovine liver catalase (Sigma), and 5.5 mM of the substrate N-formyl-Met-Ala-OH (For-Met-Ala, Bachem, Bubendorf, Switzerland). The PDF dilutions were chosen such that approx. 10% of the substrate was converted in 15 min.

After starting the reaction by the addition of the PDF, 100 µL aliquots were withdrawn from the reaction mixture every 5 minutes and added to 100 µL of 1 M phosphate buffer (H₃PO₄-NaOH), pH 2.66 to stop the reaction. Analysis of substrate and product was performed using the HPLC method described below. To obtain an enzymatic blank, 500 µL of the same phosphate buffer and 50 µL of diluted PDF were mixed together prior to the addition of 450 µL of the substrate solution given above.

One unit of activity (U) is defined as the amount of PDF which deformylates 1 µmol For-Met-Ala in 1 minute under the described conditions.

### Peptidase activity measurement

The determination of peptidases present in Cell Free Extracts (CFE) was performed at 30°C in a 500 µL solution containing each of the following 8 dipeptides in 0.625 mM concentration: H-Leu-Phe-OH, H-Phe-Leu-OH, H-Asp-Phe-OH, H-Phe-Asp-OH, H-Arg-Phe-OH, H-Phe-Arg-OH, H-Gly-Phe-OH, H-Phe-Gly-OH.

Peptidase activity was measured at 30°C in a total volume of 500 µL. 450 µL of a buffer composed of 110 mM MOPS/NaOH buffer (pH 7.2), 300 mM NaCl, 0.1 mg/mL bovine liver catalase (Sigma), and 0.625 mM of each dipeptide, were mixed with 50 µL of different dilutions of PDF in 20 mM MOPS/NaOH, 100 mM NaCl, pH 7.7, 10 µg/mL bovine liver catalase (Sigma), and 2 mM TCEP. The dilution was chosen such that approx. 10% of the substrate was converted in 15 min.

After starting the reaction by the addition of the PDF, 50 µL samples were withdrawn from the reaction mixture every 5 minutes and added to 150 µL methanol to stop the reaction. For the enzymatic blank, the methanol was added prior to the enzyme. Analysis of substrate and product was performed using the HPLC method described below.

### HPLC Analyses

HPLC was carried out using a stainless-steel analytical column (250 mm length, 4.6 mm internal diameter) packed with Inertsil ODS-3 material, 5 µm particle size from Alltech Applied Science (Breda, The Netherlands). The flow rate was 1 ml/min. Detection was performed at 40°C at a wavelength of 210 nm.

In the PDF deformylation activity measurement For-Met-Ala and Met-Ala were analysed using the following linear gradient of acetonitrile in 10 mM H₃PO₄:

| Time (min) | [acetonitrile] (vol/vol) |
|---|---|
| 0 | 0.1 |
| 4 | 0.1 |
| 15 | 50 |
| 15.1 | 0.1 |
| 20 | 0.1 |

The injection volume was 5 µL.

In the peptidase activity measurement Phe was analysed using the following linear gradient of acetonitrile in 10 mM H₃PO₄:

| Time (min) | [acetonitrle] (vol/vol) |
|---|---|
| 0 | 0 |
| 15 | 30 |
| 15.1 | 0 |
| 20 | 0 |

The injection volume was 20 µL.

### Determination of the protein concentration

The protein concentration was determined using the Bradford method. The reagent solution was prepared by dissolving 100 mg Coomassie brilliant blue G250 (SERVA Electrophoresis GmbH, Heidelberg, Gemany) in 46 mL of ethanol, 100 mL 85% phosphoric acid and bringing the total volume to 1000 mL with Milli-Q.

For the actual assay 50 µL of diluted sample were added to 950 µL reagent solution in a 1 mL cuvette. The incubation was performed for 10 min at room temperature. The absorbance was then determined at 595 nm. A calibration line was made from 0 to 0.25 mg/mL Bovine Serum Albumin (Sigma, Zwijndrecht, The Netherlands).

### Example 1: Isolation of PDF using affinity chromatography (reference)

*E. coli* PDF (wild type) was isolated from overproducing *E. coli* TOP10 cells containing plasmid pBADdef (Sonke et al., J. Mol. Catalysis B: Enzymatic 2004, 29(1-6), 265-277). Cells were grown at 28°C for 14-16 hours in 1.5 L LB medium containing 100 µg/mL carbenicillin. When the OD₆₂₀ₙₘ reached 0.6, PDF formation was induced by the addition of 0.02 wt % L-arabinose. Cells were harvested by centrifugation and about 14 g (wet weight) of cell paste were suspended in 40 mL buffer (20 mM HEPES/KOH, 100 mM KF, pH 7.7 supplemented with 10 µg/mL catalase from bovine liver (Sigma)). After disintegration by 10 min pulsed (15 microns amplitude; 10 sec on/off) sonication with Soniprep 150 (MSE, London, UK) at 0°C, cell free extract was obtained by centrifugation at 33,300 x g for 1 hour.

10 mL of the clear cell free extract (CFE: 6.45 mg/mL of protein) were fractionated using a 20 ml Met-Lys-Sepharose affinity column (Groche et al., Biochem. Biophys. Res. Commun. 1998, 246, 342-346) that had been equilibrated with buffer solution composed of 20 mM HEPES/KOH, 100 mM KF, 0.2 mM TCEP, pH 7.7. After washing with 42 mL of the same buffer solution to remove the unbound proteins, the PDF was eluted with 40 mL of 20 mM HEPES/KOH, 100 mM KCI, and 0.2 mM TCEP, pH 7.7. The fractions with PDF activity were concentrated by ultrafiltration using Centriprep 10 kDa filters (Millipore, Amsterdam, The Netherlands).

The activities obtained are given in the following table.

| | CFE | Purified PDF |
|---|---|---|
| Specific peptidase activity (Act_{peptidase}) | 0.23 U/mg | 0.0018 U/mg |
| Specific PDF activity (Act_{PDF}) | 229 U/mg | 1880 U/mg |
| (Act_{peptidase}) / (Act_{PDF}) | 1.10⁻³ | 9.57.10⁻⁷ |

From the activity data obtained the increase of purity with respect to peptidase activity was determined by calculating the ratio of the specific peptidase activity (Act_{peptidase}) to the specific PDF activity (Act_{PDF}) in the CFE and in the purified PDF, and dividing said ratio for the CFE by said ratio for the purified PDF. This calculation showed that the purity of the purified PDF increased 1049 times compared to the CFE.

### Example 2: Construction of the E-tag PDF

Standard molecular cloning techniques such as DNA isolation, gel electrophoresis, enzymatic restriction modifications of nucleic acids, *E. coli* transformation etc., were performed as described by Sambrook et al., 1989, "Molecular Cloning: a laboratory manual", Cold Spring Harbor Laboratories, Cold Spring Harbor, New York. However, when materials like enzymes and competent cells were supplied with a manual, these were used according to the instructions of the suppliers. Restriction enzymes, T4 DNA ligase, T4 DNA polymerase, and polynucleotide kinase were obtained from New England Biolabs, Schwalbach, Germany, and from Invitrogen, Breda, The Netherlands. Gateway cloning reagents and vectors, and PCR reagents were also from Invitrogen. Synthetic oligonucleotides were obtained from MWG-Biotech AG, Ebersberg, Germany or from Invitrogen. Adenosine triphosphate and deoxynucleoside triphosphates were purchased from Roche Applied Science, Mannheim, Germany.

Site-directed mutagenesis of the pdf-gene was effected by the approach described by Kunkel (Kunkel, T., Proc. Natl. Acad. Sci. USA 1985, 82, 488-492; Kunkel, T. et al., Methods Enzymol. 1987, 154, 367-382) using uracil containing single-stranded DNA (ssDNA).

For preparation of single-stranded DNA (ssDNA), which is required for this site-directed mutagenesis method, the *def*-gene was cloned between the *Eco*RI and *Hin*dIII sites of the phagemid pTZ18U (Mead, D. A., et al., Protein Eng. 1986, 1, 67-74). The plasmid p925C (Groche, D., Becker, A., Schlichting, I., Kabsch, W., Schultz, S., Wagner, A.F.V., Biochem. Biophys. Res. Commun. 1998, 246, 342-346), bearing the *def-*gene from *E. coli,* was digested with *Afl*II and *Hin*dIII. Subsequently the 5' overhangs were filled in using the Klenow fragment of the DNA polymerase I and dNTPs. Two fragments (271 bp and 3203 bp) were then separated by agarose gel electrophoresis. The 3203 bp fragment was isolated and religated usingT4 DNA ligase. *E coli* JM 109 was transformed with the ligated DNA and recombinant cells were selected on LB plates containing 100 µg/mL ampicillin. After overnight incubation at 37°C several colonies were picked and the plasmids were analyzed using restriction enzymes. One correct clone was named p963.

To transfer the *def*-gene from p963 to pTZ18U, both plasmids were digested with *Eco*RI and *Hin*dIII. The 565 bp fragment from p963 and the 2809 bp fragment from pTZ18U were isolated from agarose gel after electrophoresis. The fragments were mixed together and ligated using T4 DNA ligase. After transforming *E.coli* JM109 with the ligation mixture, recombinant cells were selected by plating on LB medium containing 100 µg/mL ampicillin. Positive colonies were analyzed by restriction enzyme analysis, and a correct phagemid was named p1074.

Phagemid p1074 was used to prepare ssDNA in which some thymidine residues were replaced by uracil. This was done by superinfection of *E. coli* strain CJ236 (ung⁻, dut⁻) (Bio-Rad Laboratories GmbH, Munich, Germany) harbouring p1074 with the helper phage M13KO7 (Pharmacia, Freiburg, Germany). *E. coli* strain CJ236 harbouring p1074 was grown in 2xYT medium (16 g/L Bacto-Tryptone, 10 g/L yeast extract, 5 g/L NaCl, 10mM KH₂PO₄ pH 7.5) supplemented with 30 µg/mL chloramphenicol, 150 µg/mL ampicillin and 1 mM thiamine hydrochloride) at 37°C with gently shaking (150 rpm). Then 0,5 mL of the culture (OD₅₅₀ₙₘ = 5) were diluted in 100 mL of the same medium and infected with M13KO7 at an MOI of 10 (MOI = phages per mL/cells per mL). Infected cells were grown at 37°C and 150 rpm for 20 h. (Final OD₅₅₀ₙₘ = 2.9). After removal of cells by centrifugation (17,000g, 20 min) the uracil-containing ssDNA of p1074 was isolated from the supernatant by precipitation with PEG 6000 (final concentration 5% PEG 6000, 0.625 M NaCl) and purified by extraction with phenol. The yield was 215 µg of pure ssDNA.

The uracil-containing ssDNA of p1074 was then used as template for the in vitro synthesis of the second strand using the following 5'-phosphorylated oligonucleotide as mutagenic primer:
5' - CGTCACGGTCCTCTTCTTCTTCCTCCTCTTCCTCTTCCGAAACATC - 3
[SEQ ID: No.7]
Application of SEQ ID: No.7 introduces eight additional codons for a Glu between Glu64 and Asn65 of the wild type PDF. Furthermore, it changes Asn65 to an Asp. Together, this insertion and mutation leads to the desired anionic tag in the flexible loop of the PDF.

For synthesis of this second strand, uracil-containing ssDNA of p1074 (0,1 pmol) were mixed with the 5'-phosphorylated mutagenic primer (2 pmol) in the presence of 20 mM Tris/HCl, pH 7.5; 2 mM Mg₂Cl, 50 mM NaCl in a final volume of 10 µL. Incubation was at 70°C for 3 min followed by 30 min at 37°C. Subsequently T4 DNA Ligase (New England Biolabs, 2 u), T4 DNA Polymerase (Roche Applied Science, 1 u) and 1 µL synthesis buffer (100 mM Tris/HCl pH 7.9; 50 mM MgCl₂; 20 mM dithiotreitol; 10 mM ATP and 5 mM of each dNTP) were added. Incubation conditions were 5 min at 0°C, followed by 5 min at 25°C, and finally 90 min at 37°C. The reaction was stopped by the addition of 10 µL buffer (100 mM Tris/HCl pH 8.0 100 mM EDTA), followed by the adjustment of the volume to 100 µL with water.

The obtained double-stranded DNA (dsDNA) (10 µL) was used to transform *E. coli* JM109 (ung⁺) (Bio-Rad Laboratories GmbH) which contains a proficient uracil N-glycosylase, that efficiently inactivates the uracil-containing strand of the phagemid leaving the mutated strand for replication. Cells were selected overnight at 37°C on LB plates containing 100 µg/ml ampicillin. Sixteen single colonies of total 294 obtained were analyzed for protein expression by SDS gel electrophoresis. Eight of the analyzed cells showed clear overexpression of PDF protein.

To simplify the detection of the target plasmid with the desired mutation, the mutant oligonucleotide used in the site directed mutagenesis procedure (SEQ ID: No.7) also introduced a number of silent mutations which resulted in the simultaneous generation of a new *Ava*ll restriction site (underlined). Isolated plasmid DNA from four of these eight clones were further analyzed by digestion with *Ava*ll for the presence of this newly introduced restriction site. All four plasmids showed the expected restriction pattern (1685 bp, 1492 bp, and 222 bp). Subsequently, the nucleotide sequence of a positive plasmid was verified by DNA sequencing of the complete mutated *def*-gene. This plasmid containing the E-tag *def*-gene encoding the PDF variant with the desired anionic tag in its flexible loop was named pTL7-1.

For regulated expression of the E-tag PDF in *E. coli* the mutated gene was cloned into expression vector pBAD/*Myc*-His-DEST using GATEWAY cloning technology (Invitrogen). Vector pBAD/*Myc*-His-DEST is a derivative of plasmid pBAD/*Myc*-HisC and has been constructed by insertion of an attR1-cat/ccdB-attR2 cassette at the position of the Shine-Dalgarno sequence in pBAD/*Myc*-HisC, which is located downstream of the strong and tightly controllable *ara*BAD promoter (EP1513946). The E-tag PDF gene was first amplified by PCR using oligonucleotide 5'-GGGG*ACAAGTTTGTACAAAAAAGCAGGCT*AGGAGGAATTAACCAATGTCCGTGCT TCAAGTGTTACATATTCC - 3'
[SEQ ID: No.8]
as forward primer (with in Italics - attB1 site, underlined - Shine-Dalgarno sequence, double underlined - parts complementary to the def gene, and in boldface - start codon), oligonucleotide 5'-GGGG*ACCACTTTGTACAAGAAAGCTGGGT*TTAAGCCCGGGCTTTCAGACGATCCA GTTTTTC - 3'
[SEQ ID: No.9] as reverse primer (with in italics - attB2 site, double underlined - parts complementary to the def gene, and in boldface - stop codon), and plasmid pTL7-1. The PCR reaction was performed using AccuPrime Taq DNA polymerase (1 U) and accompanying buffer (Invitrogen). The amplification reaction was started with an initial denaturation of 2 min at 95°C, followed by 30 cycles of 15 s at 95°C, 30 s at 58°C and 1 min at 68°C, with an additional cycle of 5 min at 68°C. The amplification product (605 nts.) was purified using the QlAquick PCR purification kit (Qiagen, Venlo, The Netherlands), after which this product was introduced into the pDONR^{™}201 vector via a Gateway^{®} BP recombination reaction in a vector:fragment molar ratio of 1:2. After transformation of *E. coli* TOP 10 recombinant cells were selected by plating on LB plates containing kanamycin (50 µg/mL), followed by overnight incubation at 28°C.

The mutated PDF gene on pDONR^{™} 201 was subsequently recombined to expression vector pBAD/*Myc*-His-DEST in a standard LR recombination reaction with a molar ratio of destination vector vs. entry vector of 1:2.4 (150:300 ng). After transformation of *E. coli* TOP10 recombinant cells were selected by plating on LB plates containing carbenicillin (100 µg/mL) followed by overnight incubation at 28°C. Finally, a correct clone, as established by plasmid DNA isolation and restriction enzyme analysis, was designated "pBAD/Myc-His-DEST E-tag PDF".

### Example 3: purification of PDF using IEC

The E. *coli* PDF E-tag mutant was isolated from overproducing *E. coli* TOP 10 cells containing plasmid pBAD/*Myc*-His-DEST EtagPDF (see Example 2 for construction). Cells were grown at 28°C for 14-16 hours in 1.5 L LB medium containing 100 µg/mL carbenicillin. When the OD₆₂₀ₙₘ reached 0.6, PDF expression was induced by the addition of 0.02 wt % L-arabinose. Cells were harvested by centrifugation and about 12.5 g (wet weight) of cell paste were suspended in 40 mL buffer (20 mM HEPES/KOH, 100 mM KCI, pH 7.7 supplemented with 10 µg/mL catalase from bovine liver (Sigma). After disintegration by 10 min pulsed (15 microns amplitude; 10 sec on/off) sonication with Soniprep 150 (MSE) at 0°C at 0°C, cell free extract was obtained by centrifugation at 33,300 x g for 1 hour.

1 mL of the clear cell free extract (CFE: 5 mg/mL of protein, according to Bradford reaction) was fractionated using a 1 mL Mono Q 5/50 GL column (GE Healthcare, Diegem, Belgium) that had been equilibrated with buffer solution composed of 20 mM Hepes/KOH, 1 mM TCEP, 0.1 M KCI, pH 7.7. After washing with 15 mL of the same buffer solution to remove the unbound proteins, the PDF was eluted with 60 mL of 20 mM Hepes/KOH, 1 mM TCEP, 1 M KCI, pH 7.7. The fractions with PDF activity (detected using the HPLC method described above) were concentrated using Centriprep 10 kDa filters (Millipore).

The activities obtained are given in the following table:

| | CFE | Purified PDF |
|---|---|---|
| Specific peptidase activity (Act_{peptidase}) | 0.163 U/mg | 0.00063 U/mg |
| Specific PDF activity (Act_{PDF}) | 177 U/mg | 967 U/mg |
| (Act_{peptidase})/ (Act_{PDF}) | 9.22.10⁻⁴ | 6.57.10⁻⁷ |

The data above showed that the purity of the purified PDF increased 1414 times compared to the CFE.

### Example 4 Purification of PDF using Ultrafiltration (UF)

*E. coli* PDF (wild type) was isolated from overproducing E. *coli* TOP 10 cells containing plasmid pBADdef (Sonke et al., J. Mol. Catalysis B: Enzymatic 2004, 29(1-6), 265-277). Cells were grown at 28°C for 14-16 hours in 0.5 L LB medium containing 100 µg/mL carbenicillin. About 4 g (wet weight) of cell paste were suspended in 20 mL buffer (20 mM MOPS/NaOH, 100 mM NaCl, pH 7.7 supplemented with 10 µg/mL catalase from bovine liver (Sigma)). After disintegration by 10 min pulsed (15 microns amplitude; 10 sec on/off) sonication with Soniprep 150 (MSE) at 0°C, cell free extract was obtained by centrifugation at 33,300 x g for 1 hour.

The resulting CFE had a protein concentration of 15 mg/mL and a PDF specific activity of 195 U/mg.

The purification step was performed using Centriprep centrifugal filter units with an Ultracel YM-100 membrane (Millipore). The nominal cut-off of those filters is 100 kDa.

Before loading the CFE to the Centriprep filter device, the sample was centrifuged for 2 hours at 16100 x g at 4°C, the supernatant was collected and subsequently diluted 5 times with buffer solution composed of 20 mM MOPS/NaOH, 100 mM NaCl, supplemented with 10 µg/mL catalase from bovine liver (Sigma) and 2 mM TCEP, pH 7.7.

The sample obtained was then loaded on a Centriprep YM-100 filter and centrifuged for 2.5 hours at 1000 x g at 4°C.

The protein concentration of the filtrate and retentate was determined using the Bradford method.

The PDF activity and the peptidase activity were determined as described above. The activities obtained are given in the following table:

| | CFE | Purified PDF |
|---|---|---|
| Specific peptidase activity (Act_{peptidase}) | 0.2 U/mg | 0.012 U/mg |
| Specific PDF activity (Act_{PDF}) | 195 U/mg | 321 U/mg |
| (Act_{peptidase})/ (Act_{PDF}) | 1.02.10⁻³ | 3.73.10⁻⁵ |

The data above showed that the purity of the purified PDF increased 27 times with respect to the CFE.

### Abbreviations:

BSA: bovine serum albumin (Sigma)
CFE: cell free extract; is the clear solution obtained after sonification and centrifugation of cells
For-Met-Ala: N-formyl-L-methionyl-L-alanine
For-Asp-Phe-OH: N-formyl-α-L-aspartyl-L-phenylalanine
For-Leu-Phe-OH: N-formyl-L-leucyl-L-phenylalanine
H-Arg-Phe-OH: L-arginyl-L-phenylalanine
H-Asp-Phe-OH: α-L-aspartyl-L-phenylalanine
H-Gly-Phe-OH: L-glycyl-L-phenylalanine
H-Leu-Phe-OH: L-leucyl-L-phenylalanine
H-Met-Ala-OH: L-methionyl-L-alanine
H-Phe-Arg-OH: L-phenylalanyl-L-arginine
H-Phe-Asp-OH: L-phenylalanyl-L-aspartic acid
H-Phe-Gly-OH: L-phenylalanyl-L-glycine
H-Phe-Leu-OH: L-phenylalanyl-L-leucine
HEPES: N-2-hydroxyethylpiperazine-N'-2-ethane sulphonic acid (Merck)
LB medium: For 1 litre, dissolve 10 g Bacto-Tryptone, 5 g Yeast Extract, 5 g NaCl in 1000 ml H₂O.
MOPS: 3-Morfolino-propane-sulphonic acid (Sigma)
TCEP: Tris (2-carboxyethyl) phosphine hydrochloride (Fluka) UF: Ultra filtration

## Claims

1. Method for preparing a purified peptide deformylase, comprising contacting a liquid which comprises a peptide deformylase containing an ionic tag and at least one other enzyme, with an ion exchange material to which material the peptide deformylase containing the ionic tag binds by ionic interaction, and separating the other enzyme from said peptide deformylase.

2. Method according to claim 1, wherein the ionic tag comprises an oligopeptide sequence comprising one or more amino acid residues having a carboxylate side-chain, preferably one ore more amino acid residues selected from the group of glutamate residues and aspartate residues.

3. Method according to claim 1, wherein the ionic tag comprises an oligopeptide sequence comprising one or more amino acid residues having an amine moiety preferably one or more amino acid residues selected from the group of lysine residues and arginine residues.

4. Method according to claim 2 or 3, wherein the ionic tag comprises an oligopeptide sequence selected from the group of oligoglutamates, oligoaspartates, oligolysines, oligoarginines, oligopeptides comprising one or more glutamate residues and one or more aspartate residues, and oligopeptides comprising one or more lysine residues and one or more arginine residues.

5. Method according to any of the preceding claims, wherein the ionic tag is an internal ionic tag.

6. Method according to any of the preceding claims, wherein the at least one other enzyme comprises an enzyme having peptidase activity, preferably an enzyme having aminopeptidase activity.

7. Peptide deformylase, in particular a recombinant peptide deformylase, provided with an internal ionic tag, in particular an internal ionic tag comprising an oligopeptide sequence selected from the group of oligoglutamates, oligoaspartates, oligolysines, oligoarginines, oligopeptides comprising one or more glutamate residues and one or more aspartate residues, and oligopeptides comprising one or more lysine residues and one or more arginine residues.

8. Peptide deformylase according to claim 7, wherein the ionic tag is present in a flexible loop of the peptide deformylase.

9. Peptide deformylase according to claim 7 or 8, comprising an amino acid sequence represented by Sequence ID 2, Sequence ID 4 or a homologue thereof.

10. A vector comprising a nucleic acid sequence encoding a peptide deformylase according to claim 7, 8 or 9.

11. A cell comprising a vector according to claim 10.

12. Method for preparing a purified peptide deformylase, comprising subjecting a liquid which comprises a peptide deformylase and at least one impurity to ultrafiltration, whereby the impurity is retained in the retentate and a filtrate is obtained comprising peptide deformylase.

13. Method according to claim 12, wherein the ultrafiltration comprises the use of a filter having a molecular weight cut-off in the range of 25 to 200 kDa, preferably in the range of 50 to 150 kDa.

14. Method according to any of the claims 12 or 13, wherein the impurity is an enzyme having peptidase activity, preferably having aminopeptidase activity.

15. Use of a peptide deformylase obtained in a method according to any of the claims 1-6, 12,13 or 14, a peptide deformylase according to claim 7, 8 or 9, or a cell according to claim 11 in the resolution of an N-formylated amino acid, amino acid derivative, peptide, or peptide derivative in peptide synthesis, or in the enantioselective formylation of an α-amino nitrile.


SEQUENCE LISTING

<110>  DSM IP ASSETS B.V.

<120>  Preparation of purified peptide deformylase

<130>  26686ep-po

<160>  9

<170>  PatentIn version 3.3

<210>  1
<211>  516
<212>  DNA
<213>  Artificial

<220>
<223>  exemplary sequence encoding PDF + ionic tag, shortest version


<220>
<221>  CDS
<222>  (1)..(516)

<220>
<221>  sig_peptide
<222>  (193)..(201)

<220>
<221>  misc_feature
<222>  (193)..(201)
<223>  n is a, c, g, or t

<400>  1
atg tcc gtg ctt caa gtg tta cat att ccg gac gag cgg ctt cgc aaa       48
Met Ser Val Leu Gln Val Leu His Ile Pro Asp Glu Arg Leu Arg Lys
1               5                   10                  15

gtt gct aaa ccg gta gaa gaa gtg aat gca gaa att cag cgt atc gtc       96
Val Ala Lys Pro Val Glu Glu Val Asn Ala Glu Ile Gln Arg Ile Val
            20                  25                  30

gat gat atg ttc gag acg atg tac gca gaa gaa ggt att ggc ctg gcg      144
Asp Asp Met Phe Glu Thr Met Tyr Ala Glu Glu Gly Ile Gly Leu Ala
        35                  40                  45

gca acc cag gtt gat atc cat caa cgt atc att gtt att gat gtt tcg      192
Ala Thr Gln Val Asp Ile His Gln Arg Ile Ile Val Ile Asp Val Ser
    50                  55                  60

nnn nnn nnn gac cgt gac gaa cgg cta gtg tta atc aat cca gag ctt      240
Xaa Xaa Xaa Asp Arg Asp Glu Arg Leu Val Leu Ile Asn Pro Glu Leu
65                  70                  75                  80

tta gaa aaa agc ggc gaa aca ggc att gaa gaa ggt tgc ctg tcg atc      288
Leu Glu Lys Ser Gly Glu Thr Gly Ile Glu Glu Gly Cys Leu Ser Ile

85                  90                  95

cct gaa caa cgt gct tta gtg ccg cgc gca gag aaa gtt aaa att cgc      336
Pro Glu Gln Arg Ala Leu Val Pro Arg Ala Glu Lys Val Lys Ile Arg
            100                 105                 110

gcc ctt gac cgc gac ggt aaa cca ttt gaa ctg gaa gca gac ggt ctg      384
Ala Leu Asp Arg Asp Gly Lys Pro Phe Glu Leu Glu Ala Asp Gly Leu
        115                 120                 125

tta gcc atc tgt att cag cat gag atg gat cac ctg gtc ggc aaa ctg      432
Leu Ala Ile Cys Ile Gln His Glu Met Asp His Leu Val Gly Lys Leu
    130                 135                 140

ttt atg gat tat ctg tca ccg ctg aaa caa caa cgt att cgt cag aaa      480
Phe Met Asp Tyr Leu Ser Pro Leu Lys Gln Gln Arg Ile Arg Gln Lys
145                 150                 155                 160

gtt gaa aaa ctg gat cgt ctg aaa gcc cgg gct taa                      516
Val Glu Lys Leu Asp Arg Leu Lys Ala Arg Ala
                165                 170


<210>  2
<211>  171
<212>  PRT
<213>  Artificial

<220>
<221>  misc_feature
<222>  (65)..(65)
<223>  The 'Xaa' at location 65 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (66)..(66)
<223>  The 'Xaa' at location 66 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (67)..(67)
<223>  The 'Xaa' at location 67 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<223>  Synthetic Construct

<400>  2

Met Ser Val Leu Gln Val Leu His Ile Pro Asp Glu Arg Leu Arg Lys
1               5                   10                  15

Val Ala Lys Pro Val Glu Glu Val Asn Ala Glu Ile Gln Arg Ile Val
            20                  25                  30

Asp Asp Met Phe Glu Thr Met Tyr Ala Glu Glu Gly Ile Gly Leu Ala
        35                  40                  45

Ala Thr Gln Val Asp Ile His Gln Arg Ile Ile Val Ile Asp Val Ser
    50                  55                  60

Xaa Xaa Xaa Asp Arg Asp Glu Arg Leu Val Leu Ile Asn Pro Glu Leu
65                  70                  75                  80

Leu Glu Lys Ser Gly Glu Thr Gly Ile Glu Glu Gly Cys Leu Ser Ile
                85                  90                  95

Pro Glu Gln Arg Ala Leu Val Pro Arg Ala Glu Lys Val Lys Ile Arg
            100                 105                 110

Ala Leu Asp Arg Asp Gly Lys Pro Phe Glu Leu Glu Ala Asp Gly Leu
        115                 120                 125

Leu Ala Ile Cys Ile Gln His Glu Met Asp His Leu Val Gly Lys Leu
    130                 135                 140

Phe Met Asp Tyr Leu Ser Pro Leu Lys Gln Gln Arg Ile Arg Gln Lys
145                 150                 155                 160

Val Glu Lys Leu Asp Arg Leu Lys Ala Arg Ala
                165                 170

<210>  3
<211>  567
<212>  DNA
<213>  Artificial

<220>
<223>  examplary sequence encoding PDF + ionic tag, longest version

<220>
<221>  CDS
<222>  (1)..(567)

<220>
<221>  sig_peptide
<222>  (193)..(252)

<220>
<221>  misc_feature
<222>  (193)..(252)
<223>  n is a, c, g, or t

<400>  3
atg tcc gtg ctt caa gtg tta cat att ccg gac gag cgg ctt cgc aaa       48
Met Ser Val Leu Gln Val Leu His Ile Pro Asp Glu Arg Leu Arg Lys
1               5                   10                  15

gtt gct aaa ccg gta gaa gaa gtg aat gca gaa att cag cgt atc gtc       96
Val Ala Lys Pro Val Glu Glu Val Asn Ala Glu Ile Gln Arg Ile Val
            20                  25                  30

gat gat atg ttc gag acg atg tac gca gaa gaa ggt att ggc ctg gcg      144
Asp Asp Met Phe Glu Thr Met Tyr Ala Glu Glu Gly Ile Gly Leu Ala
        35                  40                  45

gca acc cag gtt gat atc cat caa cgt atc att gtt att gat gtt tcg      192
Ala Thr Gln Val Asp Ile His Gln Arg Ile Ile Val Ile Asp Val Ser
    50                  55                  60

nnn nnn nnn nnn nnn nnn nnn nnn nnn nnn nnn nnn nnn nnn nnn nnn      240
Xaa Xaa Xaa Xaa Xaa Xaa Xaa Xaa Xaa Xaa Xaa Xaa Xaa Xaa Xaa Xaa
65                  70                  75                  80

nnn nnn nnn nnn gac cgt gac gaa cgg cta gtg tta atc aat cca gag      288
Xaa Xaa Xaa Xaa Asp Arg Asp Glu Arg Leu Val Leu Ile Asn Pro Glu
                85                  90                  95

ctt tta gaa aaa agc ggc gaa aca ggc att gaa gaa ggt tgc ctg tcg      336
Leu Leu Glu Lys Ser Gly Glu Thr Gly Ile Glu Glu Gly Cys Leu Ser
            100                 105                 110

atc cct gaa caa cgt gct tta gtg ccg cgc gca gag aaa gtt aaa att      384
Ile Pro Glu Gln Arg Ala Leu Val Pro Arg Ala Glu Lys Val Lys Ile
        115                 120                 125

cgc gcc ctt gac cgc gac ggt aaa cca ttt gaa ctg gaa gca gac ggt      432
Arg Ala Leu Asp Arg Asp Gly Lys Pro Phe Glu Leu Glu Ala Asp Gly
    130                 135                 140

ctg tta gcc atc tgt att cag cat gag atg gat cac ctg gtc ggc aaa      480
Leu Leu Ala Ile Cys Ile Gln His Glu Met Asp His Leu Val Gly Lys
145                 150                 155                 160

ctg ttt atg gat tat ctg tca ccg ctg aaa caa caa cgt att cgt cag      528
Leu Phe Met Asp Tyr Leu Ser Pro Leu Lys Gln Gln Arg Ile Arg Gln
                165                 170                 175

aaa gtt gaa aaa ctg gat cgt ctg aaa gcc cgg gct taa                  567
Lys Val Glu Lys Leu Asp Arg Leu Lys Ala Arg Ala
            180                 185


<210>  4

<211>  188
<212>  PRT
<213>  Artificial

<220>
<221>  misc_feature
<222>  (65)..(65)
<223>  The 'Xaa' at location 65 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (66)..(66)
<223>  The 'Xaa' at location 66 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (67)..(67)
<223>  The 'Xaa' at location 67 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (68)..(68)
<223>  The 'Xaa' at location 68 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (69)..(69)
<223>  The 'Xaa' at location 69 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (70)..(70)
<223>  The 'Xaa' at location 70 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (71)..(71)
<223>  The 'Xaa' at location 71 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (72)..(72)
<223>  The 'Xaa' at location 72 stands for Lys, Asn, Arg, Ser, Thr, Ile,

Met, Glu, Asp, Gly, Ala, Val, Gln, His, Pro, Leu, Tyr, Trp, Cys,
or Phe.

<220>
<221> misc_feature
<222> (73)..(73)
<223> The 'Xaa' at location 73 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (74)..(74)
<223> The 'Xaa' at location 74 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (75)..(75)
<223> The 'Xaa' at location 75 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (76)..(76)
<223> The 'Xaa' at location 76 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (77)..(77)
<223> The 'Xaa' at location 77 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (78)..(78)
<223> The 'Xaa' at location 78 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (79)..(79)
<223> The 'Xaa' at location 79 stands for Lys, Asn, Arg, Ser, Thr, Ile,
Met, Glu, Asp, Gly, Ala, Val, Gln, His, Pro, Leu, Tyr, Trp, Cys,
or Phe.

<220>
<221> misc_feature
<222> (80)..(80)
<223> The 'Xaa' at location 80 stands for Lys, Asn, Arg, Ser, Thr, Ile,
Met, Glu, Asp, Gly, Ala, Val, Gln, His, Pro, Leu, Tyr, Trp, Cys,

or Phe.

<220>
<221>  misc_feature
<222>  (81)..(81)
<223>  The 'Xaa' at location 81 stands for Lys, Asn, Arg, Ser, Thr, Ile,
      Met, Glu, Asp, Gly, Ala, Val, Gln, His, Pro, Leu, Tyr, Trp, Cys,
      or Phe.

<220>
<221>  misc_feature
<222>  (82)..(82)
<223>  The 'Xaa' at location 82 stands for Lys, Asn, Arg, Ser, Thr, Ile,
      Met, Glu, Asp, Gly, Ala, Val, Gln, His, Pro, Leu, Tyr, Trp, Cys,
      or Phe.

<220>
<221>  misc_feature
<222>  (83)..(83)
<223>  The 'Xaa' at location 83 stands for Lys, Asn, Arg, Ser, Thr, Ile,
      Met, Glu, Asp, Gly, Ala, Val, Gln, His, Pro, Leu, Tyr, Trp, Cys,
      or Phe.

<220>
<221>  misc_feature
<222>  (84)..(84)
<223>  The 'Xaa' at location 84 stands for Lys, Asn, Arg, Ser, Thr, Ile,
      Met, Glu, Asp, Gly, Ala, Val, Gln, His, Pro, Leu, Tyr, Trp, Cys,
      or Phe.

<220>
<223>  Synthetic Construct

<400>  4

Met Ser Val Leu Gln Val Leu His Ile Pro Asp Glu Arg Leu Arg Lys
1               5                   10                  15

Val Ala Lys Pro Val Glu Glu Val Asn Ala Glu Ile Gln Arg Ile Val
            20                  25                  30

Asp Asp Met Phe Glu Thr Met Tyr Ala Glu Glu Gly Ile Gly Leu Ala
        35                  40                  45

Ala Thr Gln Val Asp Ile His Gln Arg Ile Ile Val Ile Asp Val Ser
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Asp Arg Asp Glu Arg Leu Val Leu Ile Asn Pro Glu
                85                  90                  95

Leu Leu Glu Lys Ser Gly Glu Thr Gly Ile Glu Glu Gly Cys Leu Ser
            100                 105                 110

Ile Pro Glu Gln Arg Ala Leu Val Pro Arg Ala Glu Lys Val Lys Ile
        115                 120                 125

Arg Ala Leu Asp Arg Asp Gly Lys Pro Phe Glu Leu Glu Ala Asp Gly
    130                 135                 140

Leu Leu Ala Ile Cys Ile Gln His Glu Met Asp His Leu Val Gly Lys
145                 150                 155                 160

Leu Phe Met Asp Tyr Leu Ser Pro Leu Lys Gln Gln Arg Ile Arg Gln
                165                 170                 175

Lys Val Glu Lys Leu Asp Arg Leu Lys Ala Arg Ala
            180                 185

<210>  5
<211>  534
<212>  DNA
<213>  Artificial

<220>
<223>  preferred sequence encoding PDF + ionic tag

<220>
<221>  CDS
<222>  (1)..(534)

<220>
<221>  sig_peptide
<222>  (193)..(219)

<400>  5
atg tcc gtg ctt caa gtg tta cat att ccg gac gag cgg ctt cgc aaa       48
Met Ser Val Leu Gln Val Leu His Ile Pro Asp Glu Arg Leu Arg Lys
1               5                   10                  15

gtt gct aaa ccg gta gaa gaa gtg aat gca gaa att cag cgt atc gtc       96
Val Ala Lys Pro Val Glu Glu Val Asn Ala Glu Ile Gln Arg Ile Val
            20                  25                  30

gat gat atg ttc gag acg atg tac gca gaa gaa ggt att ggc ctg gcg      144
Asp Asp Met Phe Glu Thr Met Tyr Ala Glu Glu Gly Ile Gly Leu Ala
        35                  40                  45

gca acc cag gtt gat atc cat caa cgt atc att gtt att gat gtt tcg      192

Ala Thr Gln Val Asp Ile His Gln Arg Ile Ile Val Ile Asp Val Ser
    50                  55                  60

gaa gag gaa gag gag gaa gaa gaa gag gac cgt gac gaa cgg cta gtg       240
Glu Glu Glu Glu Glu Glu Glu Glu Glu Asp Arg Asp Glu Arg Leu Val
65                  70                  75                  80

tta atc aat cca gag ctt tta gaa aaa agc ggc gaa aca ggc att gaa       288
Leu Ile Asn Pro Glu Leu Leu Glu Lys Ser Gly Glu Thr Gly Ile Glu
                85                  90                  95

gaa ggt tgc ctg tcg atc cct gaa caa cgt gct tta gtg ccg cgc gca       336
Glu Gly Cys Leu Ser Ile Pro Glu Gln Arg Ala Leu Val Pro Arg Ala
            100                 105                 110

gag aaa gtt aaa att cgc gcc ctt gac cgc gac ggt aaa cca ttt gaa       384
Glu Lys Val Lys Ile Arg Ala Leu Asp Arg Asp Gly Lys Pro Phe Glu
        115                 120                 125

ctg gaa gca gac ggt ctg tta gcc atc tgt att cag cat gag atg gat       432
Leu Glu Ala Asp Gly Leu Leu Ala Ile Cys Ile Gln His Glu Met Asp
    130                 135                 140

cac ctg gtc ggc aaa ctg ttt atg gat tat ctg tca ccg ctg aaa caa       480
His Leu Val Gly Lys Leu Phe Met Asp Tyr Leu Ser Pro Leu Lys Gln
145                 150                 155                 160

caa cgt att cgt cag aaa gtt gaa aaa ctg gat cgt ctg aaa gcc cgg       528
Gln Arg Ile Arg Gln Lys Val Glu Lys Leu Asp Arg Leu Lys Ala Arg
                165                 170                 175

gct taa                                                               534
Ala

<210>  6
<211>  177
<212>  PRT
<213>  Artificial

<220>
<223>  Synthetic Construct

<400>  6

Met Ser Val Leu Gln Val Leu His Ile Pro Asp Glu Arg Leu Arg Lys
1               5                   10                  15

Val Ala Lys Pro Val Glu Glu Val Asn Ala Glu Ile Gln Arg Ile Val
            20                  25                  30

Asp Asp Met Phe Glu Thr Met Tyr Ala Glu Glu Gly Ile Gly Leu Ala
        35                  40                  45

Ala Thr Gln Val Asp Ile His Gln Arg Ile Ile Val Ile Asp Val Ser
    50                  55                  60

Glu Glu Glu Glu Glu Glu Glu Glu Glu Asp Arg Asp Glu Arg Leu Val
65                  70                  75                  80

Leu Ile Asn Pro Glu Leu Leu Glu Lys Ser Gly Glu Thr Gly Ile Glu
                85                  90                  95

Glu Gly Cys Leu Ser Ile Pro Glu Gln Arg Ala Leu Val Pro Arg Ala
            100                 105                 110

Glu Lys Val Lys Ile Arg Ala Leu Asp Arg Asp Gly Lys Pro Phe Glu
        115                 120                 125

Leu Glu Ala Asp Gly Leu Leu Ala Ile Cys Ile Gln His Glu Met Asp
    130                 135                 140

His Leu Val Gly Lys Leu Phe Met Asp Tyr Leu Ser Pro Leu Lys Gln
145                 150                 155                 160

Gln Arg Ile Arg Gln Lys Val Glu Lys Leu Asp Arg Leu Lys Ala Arg
                165                 170                 175

Ala

<210>  7
<211>  46
<212>  DNA
<213>  Artificial

<220>
<223>  primer

<400>  7
cgtcacggtc ctcttcttct tcctcctctt cctcttccga aacatc                    46

<210>  8
<211>  73
<212>  DNA
<213>  Artificial

<220>
<223>  primer

<400>  8
ggggacaagt ttgtacaaaa aagcaggcta ggaggaatta accaatgtcc gtgcttcaag      60

tgttacatat tcc                                                          73



<210>  9
<211>  62
<212>  DNA
<213>  Artificial

<220>
<223>  primer

<400>  9
ggggaccact ttgtacaaga aagctgggtt taagcccggg ctttcagacg atccagtttt      60

tc                                                                      62